# EUROPEAN PATENT SPECIFICATION

(11) **EP 2 126 562 B1**
(45) Date of publication and mention of the grant of the patent: **31.12.2014**
(21) Application number: 08730378.0
(22) Date of filing: 21.02.2008
(51) Int. Cl.: G01N 33/574

(54) **CLINICAL INTERVENTION DIRECTED DIAGNOSTIC METHODS**
AUF KLINISCHE EINGRIFFE GERICHTETE DIAGNOSTISCHE VERFAHREN
PROCEDE DIAGNOSTIQUE DESTINE A UNE INTERVENTION CLINIQUE

(30) Priority: 23.02.2007 US 903038 P; 17.08.2007 US 840777
(43) Date of publication of application: 02.12.2009
(73) Proprietor: Physicians Choice Laboratory Services, LLC, Rock Hill, SC 29730 (US)
(72) Inventor: SHUBER, Anthony P., Mendon, Massachusetts 01756 (US); CHIU, Eugene, Auburndale, Massachusetts 02466 (US)
(74) Representative: Kirkham, Nicholas Andrew
(86) International application number: PCT/US2008/054563
(87) International publication number: WO 2008/103815

(56) References cited:
- US-A- 5 324 634
- US-A1- 2003 082 652
- US-A1- 2006 281 093
- GOHJI ET AL: "Prognostic significance of circulating matrix metalloproteinase-2 to tissue inhibitor of metalloproteinases-2 ratio in recurrence of urothelial cancer after complete resection.", CANCER RESEARCH, vol. 56, no. 14, 1 July 1996 (1996-07-01), pages 3196-3198, XP55000960, ISSN: 0008-5472
- DURKAN, G.C., ET AL: "Alteration in Urinary Matrix Metalloproteinase-9 to Tissue Inhibitor of Metalloproteinase-1 Ratio Predicts Recurrence in Nonmuscle-invasive Bladder Cancer", CLINICAL CANCER RESEARCH, vol. 9, July 2003 (2003-07), pages 2576-2582, XP002643606,
- KAO-PENG GUAN ET AL: "Serum levels of endostatin and matrix metalloproteinase-9 associated with high stage and grade primary transitional cell carcinoma of the bladder.", UROLOGY, vol. 61, no. 4, 1 April 2003 (2003-04-01), pages 719-723, XP55000965, ISSN: 0090-4295
- Fernandez, C.A., et al: "Development of a Diagnostic Test for Monitoring Bladder Cancer Recurrence Using Urinary Matrix Metalloproteinases (MMPs) as Clinical Biomarkers", Internet , August 2007 (2007-08), XP002643608, Retrieved from the Internet: URL:http://www.predictivebiosci.com/pdfs/p osters_abstracts/CHI_%20Biomarker_Poster_0 8_2007.pdf [retrieved on 2011-06-15]
- FERNANDEZ C A ET AL: "A Novel Approach to Using Matrix Metalloproteinases for Bladder Cancer", JOURNAL OF UROLOGY, LIPPINCOTT WILLIAMS & WILKINS, BALTIMORE, MD, US, vol. 182, no. 5, 1 November 2009 (2009-11-01), pages 2188-2194, XP026677719, ISSN: 0022-5347, DOI: DOI:10.1016/J.JURO.2009.07.032 [retrieved on 2009-09-16]
- GALLEY, H.F.: "Solid as a ROC", BRITISH JOURNAL OF ANAESTHESIA, vol. 93, no. 5, 2004, pages 623-626,

## Description

### Priority Application

This application claims priority to U.S. Application No. 11/840,777, filed on August 17, 2007 and claims benefit to U.S. Serial No. 60/903,038, filed on February 23, 2007.

### Background of the Invention

Standard screening assays are used by clinicians to assess the current health status of patients and to provide insight into the patient's risk of having a particular disease or condition. Screening assays generally employ a threshold above which a patient is screened as "positive" for the indicated disease and below which the patient is screened as "negative" for the indicated disease. Thresholds in screening assays generally are chosen in order to maximize the number of patients who will receive further intervention in the form of diagnostic monitoring or therapy. However, all screening assays result in false positive and false negative determinations. This means that there is a portion of the screened patient population who are screened positive and prescribed further intervention who, in fact, are negative and do not need further intervention. There are also patients who are screened negative but who are actually positive and require accelerated or more significant (i.e. relative to standard of care) intervention. In standard screening assays, patients cannot be unambiguously placed into any clinical category. Thus, there is always a population of patients (false negatives and false positives) who are referred from improper follow-up due to the ambiguity inherent in screening.

An area in which this ambiguity has particular significance for patients is that of recurrence monitoring in cancer. Cancer patients who have been successfully treated must be concerned that either a primary tumor will recur or a secondary tumor will develop as a result of chemotherapy and radiation used to eradicate the original cancer. Screening those patients for recurrence is important, as many recurrent cancers can be treated with minimal intervention if caught early enough. Thus, most cancer survivors are monitored on intervals that depend primarily upon the type of cancer for which they were originally treated and the type of original treatment they received. Screening assays for recurrent cancer apply the same statistical criteria as do cholesterol screening and other common assays. Thus, there are a significant number of patients whose assay scores are ambiguous because they are at or near the limit of a range for "normal" samples. Those patients typically are required to continue screening and perhaps are even subjected to prophylaxis that may be unnecessary but is prescribed by the prevailing standard of care. Moreover, patients who are at increased risk of recurrence often are not identified as such and therefore are not provided with increased surveillance that may be necessary to effect early detection of recurrence. There is, therefore, a need in the art to provide screening assays that eliminate as many ambiguous results as possible, thereby limiting the number of patients who must endure unnecessary procedures and optimizing identification of patients who would certainly benefit from continual monitoring and/or intervention.

### Summary of the Invention

The invention provides methods for assessing the clinical status of a patient. In particular, the invention provides methods for identifying the presence of or likelihood of cancer or cancer recurrence. In practice, methods of the invention provide the ability to screen patients into one of three distinct clinical categories. Based upon measurement of clinically-relevant biomarkers in a sample obtained from a patient, the invention allows the unambiguous identification of patients who are not at risk for or do not have the relevant cancer, the unambiguous identification of patients at increased risk or who have the cancer; and the identification of patients who should receive standard of care treatment and/or monitoring. Use of the invention maximizes the number of patients who will receive accelerated intervention or monitoring and minimizes those patients who will receive unnecessary standard of care or accelerated intervention or monitoring.

Methods of the invention are particularly useful in the clinical assessment of cancer recurrence. Practice of the invention allows the unambiguous identification of patients who are not at risk for cancer recurrence or who do not have recurrent cancer, and those who are at heightened risk of recurrence or who have recurrent cancer. Thus, practice of the invention allows a clinician to differentially stratify patients in order to reduce or eliminate monitoring/treatment for an entire group of patients. The invention also provides means to identify those patients requiring increased monitoring and/or intervention. Patients who do not fit into either of those categories receive standard of care monitoring and/or intervention. Practice of the invention allows a clinician to eliminate patients from further diagnostic or therapeutic intervention who are at no risk of cancer and to increase intervention for patients who are at increased risk. Methods of the invention can be repeated longitudinally in order to provide an ongoing assessment of patient health. At each point of testing, a patient is placed into one of the three categories mentioned above - those at no current risk, those at heightened current risk, and those in between.

According to the invention, an amount of a biomarker is measured in a patient sample and then is compared to one or more threshold levels of the same biomarker. Based upon the comparison of the patient's biomarker level and the threshold values, the patient is assigned to one of three groups. The first group consists of patients who have biomarker levels that are below a threshold level that is unambiguously-associated with non-recurrence of a cancer with which the biomarker is associated. The second group consists of patients who have biomarker levels that are unambiguously-associated with recurrence of the cancer. The final group of patients have biomarker levels that are between the two thresholds and therefore are subject to standard screening assay uncertainty in their diagnosis. According to methods of the invention, patients in the first group can forego additional. current monitoring, prophylactic, or therapeutic intervention with respect to the disease. The second group should have increased monitoring relative to the standard of care; and the third group is proposed to be subject to the standard of care for recurrence monitoring for the disease in question.

Thus, the invention provides a way of stratifying patients so as to provide increased vigilance to those who need it, spare those who don't need additional monitoring at any given point in time, and apply the standard of care to the remaining patients. In addition to providing increased certainty with respect to significant portions of the population, the invention allows cost savings and reduced risk of a misdiagnosis due to ambiguities associated with typical screening assays.

In another embodiment not being part of the invention, a single threshold is used in order to identify a subset of patients who unambiguously do not have recurrent disease. For example, a biomarker indicative of recurrent disease is obtained from a patient. The amount of the biomarker in the patient sample is compared to a threshold below which no recurrence is expected. Those patients can then be informed unambiguously that, at that time, they do not need to follow additional monitoring, therapy, or screening under the normal standard of care for recurrent disease. The remaining patients are provided with standard of care screening, monitoring, and or therapy. The follow-on monitoring procedure may be an imaging procedure or an invasive procedure. Examples of imaging procedures include, but are not limited to, PET scan, MRI, CT scan, ultrasonography, Doppler study, nuclear scan, and X-ray. The invasive procedure may be surgical or medical. Examples of surgical procedures include, but are not limited to, biopsy, resection, excision, or aspiration. Examples of medical procedures include, but are not limited to, cystoscopy, bronchoscopy, laparoscopy, colonoscopy, sigmoidoscopy, laryngoscopy, angiography, or catheterization. Follow-on therapeutic procedures may be chemotherapy, surgery, radiation or hormonal therapy.

Methods of the invention can be performed with respect to any disease, but are especially applicable to diseases having a high rate of recurrence, such as cancer. The thresholds used to classify patients for further screening or monitoring can be obtained from the literature, from known indications, or can be derived empirically. The particular threshold chosen will depend upon the particular biomarker being assayed. For certain markers, thresholds can be established based upon known sensitivity and specificity data. For example, biomarker-based screens using high specificity (e.g., 100%) cutoff values to provide high positive predictive value (e.g., 100% PPV) may be used to determine with certainty whether a patient (e.g., a cancer patient) needs to undergo further diagnostic or therapeutic procedures. Biomarker-based screens using high sensitivity (e.g., 95-100%) cutoff values to provide high negative predictive values (e.g., 95-100% NPV) may be used to determine with certainty whether a patient (e.g., a cancer patient) can forgo further diagnostic or therapeutic procedures. In certain circumstances, results can incorporate other relevant information about the patient to fine-tune diagnostic advice. Sensitivity represents how well an assay identifies the proportion of true positives for a disease in the screening assay and can be expressed as the number of true positives divided by the combination of true positives and false negatives. Specificity represents the proportion of true negative cases in the population and can be expressed as the number of true negatives divided by the number of true negatives plus the number of false positives.

Methods of the invention are useful to improve the performance characteristics of existing clinical screening assays and algorithms. For example, standard screening assays can be transformed to render unambiguous results with respect to patient populations that either do not require further monitoring or that require increased monitoring. Methods of the invention are applicable to any clinical screening algorithm in which there are thresholds that have been or can be identified as described herein in order to categorize patients with respect to a particular disease-associated biomarker or set of biomarkers. Multiple biomarkers are useful in the context of the invention in order to provide additional sensitivity and specificity around thresholds for categorizing patients.

Due to the principles involved, the invention is applicable to monitoring recurrence in any disease, either in symptomatic or asymptomatic patient populations. For example, the invention can be applied to patients who previously have had cancer, an infectious disease, diabetes, neurologic disease, metabolic disease, and others. Examples of biomarkers associated with cancer include matrix metalloproteinase (MMP), neutrophil gelatinase-associated lipocalin (NGAL), MMP/NGAL complex, thymosin □15, thymosin □16, collagen like gene (CLG) product, prohibitin, glutathione-S-transferase, beta-5-tubulin, ubiquitin, tropomyosin, Cyr61, cystatin B, chaperonin 10, and profilin. Examples of MMPs include, but are not limited to, MMP-2, MMP-9, MMP9/NGAL complex, MMP/TIMP complex, MMP/TIMP1 complex, ADAMTS-7 or ADAM-12, among others. Also, the patient sample from which a biomarker is obtained is immaterial to the functioning of the invention. Preferred sample sources include blood, serum, sputum, stool, saliva, urine, cerebral spinal fluid, breast nipple aspirate, and pus.

The invention can also be used to monitor the progress of therapeutic treatment. In that context, a biomarker indicative of a disease is obtained from a patient being treated for the associated disease. The amount of the biomarker is compared to a predetermined threshold below which a patient can be unambiguously identified as not requiring further therapeutic intervention at that time. In another embodiment, a second threshold can be applied to identify patients having levels of the selected biomarker that unambiguously indicate that therapy should be increased and/or intensified. Patients between the two thresholds are indicated to receive continued standard of care therapy.

Methods of the invention can be used on patients known to have a disease, or can be used to screen healthy subjects on a periodic basis. A subject can be screened for one or more diseases simultaneously. Screening can be done on a regular basis (e.g., weekly, monthly, annually, or other time interval); or as a one time event. The outcome of the analysis may be used to alter the frequency and/or type of screening, diagnostic and/or treatment protocols. Different conditions can be screened for at different time intervals and as a function of different risk factors (e.g., age, weight, gender, history of smoking, family history, genetic risks, exposure to toxins and/or carcinogens etc., or a combination thereof). The particular screening regimen that is used in connection with the invention and the particular biomarkers that are selected are determined at the discretion of the physician or technician.

Threshold values for any particular biomarker and associated disease are determined by reference to literature or standard of care criteria or may be determined empirically. In a preferred embodiment of the invention, thresholds for use in the invention are determined as levels of a particular biomarker used in connection with the invention below which there is approximately 100% negative predictive value. In other words, patients having values below the threshold are not expected to have the disease for which the screen is being conducted and can unambiguously be determined not to need further intervention at that time. In the dual threshold embodiment, a second threshold is determined that has approximately 100% positive predictive value. Biomarker levels above that threshold are unambiguously associated with the need for further intervention. Obviously, in certain disease states, the negative and positive predictive values are reversed, such that a level below the lower threshold indicates the need for further monitoring and a value above the higher threshold signals an unambiguous determination that the patient need not be further monitored. Moreover, with certain biomarkers, positive and negative predictive values do not have to be 100%, but can be something less than that depending upon other factors, such as the patients genetic history or predisposition, overall health, the presence or absence of other markers for diseases, etc. While, unambiguous classification according to the invention ideally means 100% predictive value, there is an acceptable range below 100%, ideally between 95% and 100%. According to the invention, unambiguous classification can also be based upon the recognition of an association of a disease state with a particular biomarker level, levels, or range of levels. Finally, unambiguous classification can be based upon a predetermined level of a marker above or below which all affected or unaffected patients are found. For example, in one embodiment, the invention contemplates determining the level of a biomarker in a patient and classifying the patient as having a disease if the patient's biomarker level is above a level present in all patients having the disease.

Further aspects and features of the invention will be apparent upon inspection of the following detailed description thereof.

### Description of the Drawings

Figure 1 is a curve showing levels of MMP-9 obtained from cancer patients and controls.
Figure 2 is an overlay showing the MMP-9 data discussed below on the overlapping curves exemplifying cutoffs for identifying disease recurrence according to methods of the invention.33

### Detailed Description of the Invention

The invention provides for the ability to unambiguously place patients into different populations for the screening of recurrent cancer. According to the invention, patients having a biomarker associated with recurrent cancer that falls below a first predetermined threshold are unambiguously placed in a group that does not need standard of care therapy, monitoring, or other intervention. Those patients who have biomarker levels that exceed a second predetermined threshold are placed into a group that receives accelerated or increased therapy, monitoring, or other intervention. Those patients between the thresholds are provided with normal standard of care therapy, monitoring or other intervention. In one aspect, a single threshold is used in order to rule out a subset of patients who do not need to undergo continued therapy, monitoring or other intervention.

The invention incorporates the notion that there are relevant levels of disease-associated biomarkers that allow one to predict recurrence or non-recurrence unambiguously. By that it is meant that subsets of patients can be excluded from further monitoring or provided with increased vigilance with between about 95% and about 100% certainty with respect to the outcome.

Patients having been treated for a cancer for which there is a predictive biomarker will fall within a continuum of values with respect to recurrence. At the ends of that continuum, one can predict with certainty or near-certainty whether the patient will develop recurrent cancer. Thus, patients who fall below a first threshold can be determined to have not developed recurrent cancer with a negative predictive probability of 100%; whereas those patients who have values that are greater than a second threshold can be determined to have recurrent cancer with a positive predictive probability of 100%. One can select the two threshold lines to reflect any percentage of sensitivity and specificity that are desired, but ideally, thresholds are set so that the negative predictive value of the lower threshold is greater than about 95% and the positive predictive value of the higher threshold is greater than about 95%. Negative predictive value is the ratio of true negatives to true negatives plus false negatives. Positive predictive value is the ratio of true positives to true positives plus false positives.

There are other methods for determining thresholds for use in the invention, including reference to standard values in the literature or associated standards of care. However, the exact values selected are immaterial because what matters is the association of the threshold with high predictive value with respect to the patients falling outside the thresholds.

Similarly, the biomarker chosen is immaterial to the operation of the invention as long as the marker is associated with the disease for which screening is being conducted. Some biomarkers that have been associated with disease include nucleic acid markers (including but not limited to K-ras, K-ras2, APC, DCC, TP53, PRC1, NUSAP1, CAPZ, PFKP, EVER1, FLT1, ESPL1, AKAP2, CDC45L, RAMP, SYNGR2, NDRG1, ZNF533, and hypermethylated nucleic acid), proteins and peptides, carbohydrates, sugars, glycans, lipids, hormones (e.g., antidiuretic hormone (ADH), Adrenocorticotrophic hormone (ACTH), growth hormone(GH), follicle stimulating hormone (FSH), luteinizing hormone (LH), estrogen (estradiol, estrone, estriol), progesterone, testosterone, dihydrotestosterone (DHT), inhibin, somatotropin, dehydroepiandrostenedione (DHEA), somatostatin, glucagon, insulin, thyrotropin, thyroid stimulating hormone (TSH), thyroxin, parathyroid hormone, corticotropin, cortisol, corticosteron, aldosterone, epinephrine, norepinephrine, prolactin, vasopressin, oxytocin, melanocyte stimulating hormone (MSH)), growth factors (e.g., granulocyte-colony stimulating factor (G-CSF), granulocyte- macrophage colony stimulating factor (GM-CSF), nerve growth factor (NGF), neurotrophins, platelet-derived growth factor (PDGF), erythropeitin (EPO), thrmobopoeitin (TPO), myostatin (GDF-8), growth differentiation factor (GDF-9), basic fibroblast growth factor (bFGF or FGF2), acidic fibroblast growth factor, epidermal growth factor (EGF), hepatocyte growth factor (HGF), human stem cell factor (SCF), tumor necrosis factor (TNF), tumor necrosis factor-β (TNF-β), tumor necrosis factor-α (TNF-α), vascular endothelial growth factor (VEGF), transforming growth factor-β (TGF-β), transforming growth factor-α (TGF-α), insulin-like growth factor-I (IGF-II), insulin-like growth factor-II (IGF-II), and colony stimulating factor (CSF)), cytokines (e.g., IL-1α, IL-1β, IL-2, IL-3, IL-4, IL-5, IL-6, IL-7, IL-8, IL-9, IL-10, IL-11, IL-12, IL-13 , IFN-α, IFN-β, and IFN-γ), proteins (e.g., Matrix metalloproteinases (MMPs) such as MMP2, MMP9, neutrophil gelatinase-associated lipocalin (NGAL), MMP/NGAL complex, thymosin β15, thymosin β16, collagen like gene (CLG) product, prohibitin, glutathione-S-transferase, beta-5-tubulin, ubiquitin, tropomyosin, Cyr61, cystatin B, chaperonin 10, profilin, Alpha-fetoprotein, Carcinoembryonic antigen, Epidermal growth factor receptor, Kallikrein 3 (prostate specific antigen), Vascular endothelial growth factor A, VEGF, Albumin, CA 125, Calcitonin, Chromogranin A (parathyroid secretory protein 1), Corticotropin-lipotropin contains ACTH, Estrogen receptor 1, Gastrin, Progesterone receptor, Prolactin, S100 alpha chain, Somatostatin, Thyroglobulin, V-erb-b2, Her2/neu, Antigen identified by monoclonal antibody Ki-67, B-cell CLUlymphoma 2, BCL2-associated X protein, Beta-2-microglobulin, Breast cancer 1 early onset, BRCA1, CA 15.3, CA 19.9, Cadherin 1 type 1 E-cadherin (epithelial), Caspase 3, CD44 antigen, Cellular tumor antigen p53, Coagulation factor II, prothrombin, Colony stimulating factor 2 (granulocyte-macrophage), Colony stimulating factor 3 (granulocyte), C-reactive protein, Cyclin D1, Cyclin-dependent kinase inhibitor 1, p21, Erythropoietin, Fibrinogen alpha/alpha-E chain, Follicle-stimulating hormone, Gamma enolase, Insulin, Interferon gamma, Interleukin 2, Interleukin 6, k-ras, Neprilysin, CD 10, Transferrin, Trypsin, Tumor necrosis factor (TNF-alpha), Tumor necrosis factor receptor superfamily member 6, fas, Von Willebrand Factor, Chemokine, Chitinase-3 like protein 1, YKL-40, Choriogonadotropin beta chain, Colony stimulating factor 1 (macrophage), Haptoglobin-1, Hepatocyte growth factor, Inhibin, Interferon-alpha/beta receptor alpha chain, Interferon-alpha/beta receptor beta chain, Kallikrein 10, Kallikrein 11, Kallikrein 6, Matrix metalloproteinase 3, ADAM-12, Small inducible cytokine A21 (CCL21) soluble IL-2R alpha, Somatotropin growth factor, growth hormone, Breast cancer 2 early onset, BRCA2, Catenin Beta 1, Cathepsin D, CD15, Desmin, DNA-(apurinic or apyrimidinic site) lyase, APEX, Lutropin beta chain, Luteinizing hormone, Parathyroid Hormone, Proliferating cell nuclear antigen, Tumor necrosis factor ligand superfamily member 8 (CD30 ligand), V-myc myelocytomatosis viral oncogene homolog (avian), Tumor necrosis factor ligand superfamily member 8 (CD30), 17beta-Hydroxysteroid dehydrogenase type 1 (17HSD1), Acid phosphatase prostate, Adrenomedullin, Aldolase A, bone-specific Alkaline phosphatase, Alkaline phosphatase, placental type, Alpha-1-acid glycoprotein 1, orosomucoid, Alpha-1-antitrypsin, alpha-2-H S-glycoprotein, Alp ha-2-macroglobulin, Alpha-lactalbumin, Angiogenin ribonuclease RNase A family 5, Angiopoietin 1, Angiopoietin 2, Antileukoproteinase 1, SLPI , Apolipoprotein Al, Apolipoprotein A-II, Apolipoprotein C-1, Apolipoprotein C-III, Bone sialoprotein II, Brain-derived neurotrophic factor, Breast cancer metastasis-suppressor 1, CA 27.29, CA 72-4, Cathepsin B, CC chemokine 4, HCC-4, CD44 variant V5 soluble, Ceruloplasmin, Cervical cancer 1 protooncogene protein p40, Chemokine (C-C motif) ligand 4 Small inducible cytokine A4 (CCL4),MIP-1-beta, Claudin-3, Claudin-4, Clusterin, Coagulation factor III, Coagulation factor XIII A chain, Coagulation factor XIII B chain, Collagen I c-terminal telopeptide, Complement component 3, Complement component 4, Complement component 7, Complement factor H related protein, Cyclin-dependent kinase 6, Cyclooxygenase-2, Cystatin A, Cystatin B, Cystatin C, Cytokeratin 8, Diazepam binding inhibitor, Endoglin, Endothelin 1, Epidermal growth factor, E-selectin, Ferritin H, Fibroblast growth factor 2 (basic), Fibronectin 1, Flt-3 ligand, Fms-related tyrosine kinase 1, VEGFRI, Follistatin, Fructose-bisphosphate aldolase B, Fructose-bisphosphate aldolase C, Geminin, Glucose-6-phosphate isomerase, Glypican-3, n-terminal, Growth arrest and DNA-damage-inducible alpha, Immunosuppressive acidic protein, Insulin-like growth factor 1 (somatomedin C), Insulin-like growth factor 2 (somatomedin A), Insulin-like growth factor binding protein 1, Insulin-like growth factor binding protein 2, Insulin-like growth factor binding protein 3, Intercellular Adhesion Molecule 1, Interferon alpha 1, Interleukin 1 alpha, Interleukin 1 beta, Interleukin 10, Interleukin 12A, Interleukin 16, Interleukin 5, Interleukin 6 receptor, Interleukin 6 signal transducer, Interleukin 7, Interleukin 8, Interleukin 9, Interleukin-1 receptor antagonist protein, IRAP, Kallikrein 14 (hK14), Kallikrein 2 prostatic, Kallikrein 5, Kallikrein 7, Kallikrein 8, Kallikrein 18, Kallikrein 8, Keratin 18, Keratin, type I cytoskeletal 19, cytokeratin 19, Kit ligand, Lactotransferrin, Leptin, L-selectin, Luteinizing hormone-releasing hormone receptor, Mac-2 Binding Protein 90K, Mammaglobin B, Mammary Serum, Antigen, Mast/stem cell growth factor receptor, , Melanoma-inhibiting activity, Membrane cofactor protein, CD46 antigen, Mesothelin, Midkine, MK-1 protein, Ep-CAM, Myoblast determination protein 1, Nerve growth factor beta, Netrin-1, Neuroendocrine secretory protein-55, Neutrophil defensin 1, Neutrophil defensin 3, Nm23-H 1, OVX1, OX40, p65 oncofetal protein, Pancreatic secretory trypsin inhibitor, TATI, Parathyroid hormone-related protein, Pcaf, P300/CBP-associated factor, Pepsinogen-1, Placental specific tissue protein 12 Plasma retinol-binding protein, Plasminogen (Contains Angiostatin), Platelet endothelial cell adhesion molecule, PECAM-1, Platelet factor 4, Platelet-derived growth factor beta polypeptide, Platelet-derived growth factor receptor alpha polypeptide, Pregnancy zone protein, Pregnancy-associated plasma protein-A, Prostate secretory protein PSP94, P-selectin, PSP94 binding protein, Pyruvate kinase, isozymes M1/M2, Riboflavin carrier protein, 100 beta chain, Secreted phosphoprotein 1, osteopontin, Serine (or cysteine) proteinase inhibitor clade B, maspin, Serine (or cysteine) proteinase inhibitor clade E, PAI-1, Serum amyloid alpha-1, Serum paraoxonase/arylesterase 1, Small inducible cytokine A14 CCL14, Small inducible cytokine A18(CCL18), MIP-4, Small inducible cytokine A2(CCL2), Small inducible cytokine A3(CCL3), Macrophage inflammatory protein 1-alpha, Small inducible cytokine B5(CXCL5), Squamous cell carcinoma antigen 1, Squamous cell carcinoma antigen 2, Survivin, Syndecan-1, synuclein-gamma, TEK tyrosine kinase endothelial, Tie-2, Tenascin, Tetranectin, TGF-beta receptor type III, Thiredoxin reductase 1, Thrombopoietin, Thrombopoietin 1, Thymidin kinase, Tissue inhibitor of metalloproteinase1, Tissue inhibitor of metalloproteinase2, Tissue-type plasminogen activator, tPA, Transferrin receptor (p90 CD71), Transforming growth factor alpha, Transforming growth factor beta 1, transthyretin, Tropomyosin 1 alpha chain (Alpha-tropomyosin), Tumor necrosis factor (ligand) superfamily member 5, CD 154, Tumor necrosis factor (ligand) superfamily member 6, Fas ligand, Tumor necrosis factor ligand superfamily member 13B, TALL-1, Tumor necrosis factor receptor superfamily member 11B, osteoprotegerin, Tumor necrosis factor receptor superfamily member 1A p60 TNF-RI p55 CD120a, TNFR1, Tumor necrosis factor receptor superfamily member 1 B, TNFR2, Urokinase plasminogen activator surface receptor, U-PAR, Vascular cell adhesion molecule 1, Vascular endothelial growth factor receptor 2, Vasoactive intestinal peptide, VEGF(165)b, Vitamin K dependent protein C, Vitronectin, and X box binding protein-1), antibodies, or any combination thereof.

### Example

Methods of the invention were applied to a cohort of healthy patients, cancer patients, and cancer-free patients presenting with a variety of other symptoms. Twenty-eight patients with cancer, 124 healthy patients, and 78 patients presenting with other symptoms were tested using methods of the invention in a blinded study. The table below shows the various groups of patients as categorized for the study.

| **Disease Indication (N)** |
|---|
| Cancer (28) |
| Healthy (124) |
| Infertility (6) |
| Hernia (6) |
| Erectile Dysfunction (14) |
| Urinary Tract Infection (2) |
| Kidney Stones (20) |
| Urinary Incontinence (11) |
| Hematuria (7) |
| Diabetes (5) |
| Bladder Obstruction (7) |

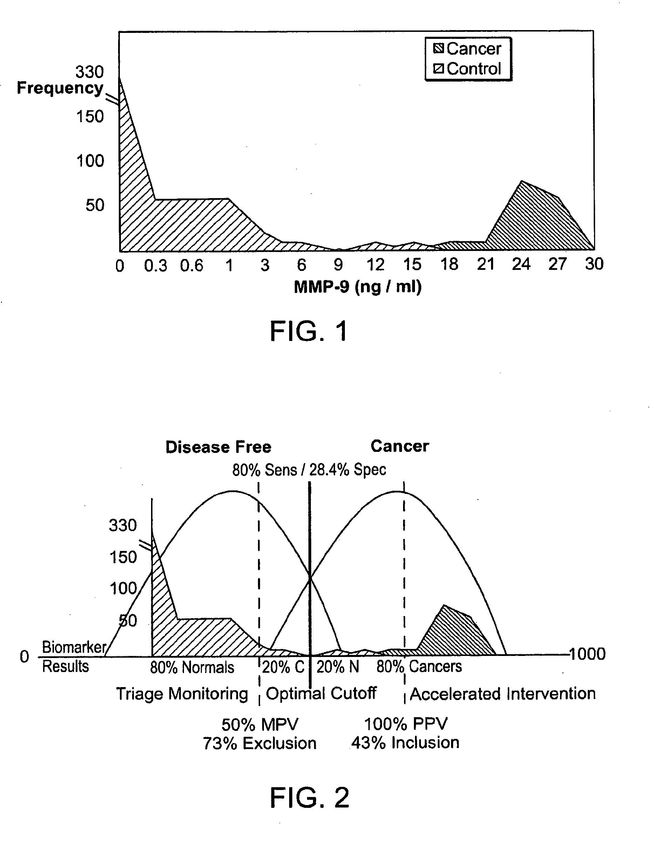

Blood samples were obtained from all patients and levels of the matrix metalloproteinase, MMP-9, were obtained using a standard ELISA. As shown below in Figure 1, differences in MMP-9 between cancer and non-cancer patients were statistically significant. Those differences enabled the acquisition of thresholds as shown in Figure 2 in which non-cancer patients were unambiguously identified with a negative predictive value of 99%. That allowed the exclusion of 73% of patients who, in standard diagnostic monitoring, would have been prescribed to receive addition (and unnecessary) screening and/or treatment. The higher threshold in Figure 2 allowed cancer patients to be unambiguously identified as requiring follow-up with a 100% positive predictive value. This resulted in 43% of patients who, in standard diagnostic monitoring, would have received only standard of care monitoring and/or therapy, but who, as a result of application of the invention, receive more aggressive monitoring and/or therapy. Patients between the thresholds receive "standard of care" treatment. The "optimal cutoff" shown in Figure 2 is the cutoff applied under current standard of care screening, which would have resulted in 16% of the disease-free patient population being diagnosed as having cancer and then being unnecessarily treated; and 14% of cancer patients being diagnosed as healthy and receiving inadequate intervention. Thus, application of the invention in order to identify unambiguous thresholds resulted in more accurate diagnosis and the ability to prescribe more effective intervention.

According to the invention, the thresholds determined for MMP-9 are used to screen bladder cancer patients into one of three groups: those who are unambiguously identified as not being at risk for recurrent disease, those at heightened risk for recurrent disease, and those whose screening results are ambiguous and should receive standard of care monitoring and/or therapy.

## Claims

1. An in vitro method for performing a clinical assessment in a patient who has previously been treated for cancer, the method comprising the steps of:
Establishing a first threshold of a biomarker determined based upon a range of levels of said biomarker obtained from a first population of patients having a primary cancer and a recurrent cancer and a second population of patients having a cancer but no recurrent cancer and below which it would be expected that between 95% and 100% of patients would be in said second population of patients;
Establishing a second threshold of said biomarker determined based upon a range of levels of said biomarker obtained from a first population of patients having a primary cancer and a recurrent cancer and a second population of patients having a cancer but no recurrent cancer above which it would be expected that between 95% and 100% of patients would be in said first population of patients;
Obtaining a level of a biomarker in a patient who has previously been treated for cancer by measuring the level of that biomarker in a sample from the patient;
Applying said level to determine whether said level is below said first threshold, indicating that no current diagnostic intervention is required or whether said level is above said second threshold, indicating that increased diagnostic intervention is required; and
Determining that said patient is in need of a standard of care diagnostic regimen if said level is between said first threshold and said second threshold.

2. The method of claim 1, wherein said biomarker is selected from the group consisting of matrix metalloproteinases (MMP), neutrophil gelatinase-associated lipocalin (NGAL), MMP/NGAL complex, thymosin βI5, thymosin βI6, collagen like gene (CLG) product, prohibitin, glutathione-S-transferase, beta-5-tubulin, ubiquitin, tropomyosin, Cyr61, cystatin B, chaperonin 10, TIMP, and profilin.

3. The method of claim 2, wherein said MMP is selected from the group consisting of MMP- 2, MMP-9, MMP9/NGAL complex, MMP/TIMP complex, MMP/TIMP1 complex, ADAMTS-7 or ADAM-12.

## Patentansprüche

1. In-vitro-Verfahren zum Durchführen einer klinischen Beurteilung bei einem Patienten, der zuvor auf Krebs behandelt wurde, wobei das Verfahren die folgenden Schritte umfasst:
Festlegen eines ersten auf der Grundlage eines Bereichs von Spiegeln des Biomarkers bestimmten Grenzwerts eines Biomarkers, der aus einer ersten Patientenpopulation mit einem primären Krebs und einem wiederkehrenden Krebs und einer zweiten Patientenpopulation mit einem Krebs, aber keinem wiederkehrenden Krebs, ermittelt wird, und unterhalb dem erwartet würde, dass zwischen 95% und 100% der Patienten in der zweiten Patientenpopulation wären;
Festlegen eines zweiten auf der Grundlage eines Bereichs von Spiegeln des Biomarkers bestimmten Grenzwerts des Biomarkers, der aus einer ersten Patientenpopulation mit einem primären Krebs und einem wiederkehrenden Krebs und einer zweiten Patientenpopulation mit einem Krebs, aber keinem wiederkehrenden Krebs, ermittelt wird, und oberhalb dem erwartet würde, dass zwischen 95% und 100% der Patienten in der ersten Patientenpopulation wären;
Ermitteln eines Spiegels eines Biomarkers bei einem Patienten, der zuvor auf Krebs behandelt wurde, durch das Messen des Spiegels jenes Biomarkers in einer Probe des Patienten;
Anwenden des Spiegels, um zu bestimmen, ob der Spiegel unter dem ersten Grenzwert liegt, was anzeigt, dass kein aktueller diagnostischer Eingriff erforderlich ist, oder ob der Spiegel über dem zweiten Grenzwert liegt, was anzeigt, dass ein verstärkter diagnostischer Eingriff erforderlich ist; und
Bestimmen, dass der Patient einen Standard eines diagnostischen Pflegeprogramms benötigt, wenn der Spiegel zwischen dem ersten Grenzwert und dem zweiten Grenzwert liegt.

2. Verfahren nach Anspruch 1, wobei der Biomarker aus der Gruppe ausgewählt ist, die besteht aus Matrix-Metalloproteinasen (MMP), neutrophilengelatinase-assoziiertem Lipocalin (NGAL), MMP/NGAL-Komplex, Thymosin β15, Thymosin β16, Collagen-ähnlichem Genprodukt (CLG-Produkt), Prohibitin, Glutathion-S-Transferase, Beta-5-Tubulin, Ubiquitin, Tropomyosin, Cyr61, Cystatin B, Chaperonin 10, TIMP und Profilin.

3. Verfahren nach Anspruch 2, wobei die MMP aus der Gruppe ausgewählt ist, die besteht aus MMP-2, MMP-9, MMP9/NGAL-Komplex, MMP/TIMP-Komplex, MMP/TIMP1-Komplex, ADAMTS-7 oder ADAM-12.

## Revendications

1. Procédé in vitro pour effectuer une évaluation clinique chez un patient qui a préalablement été traité pour le cancer, le procédé comprenant les étapes de :
- établir un premier seuil d'un biomarqueur déterminé sur la base d'une plage de taux dudit biomarqueur obtenue à partir d'une première population de patients ayant un cancer primaire et un cancer récidivant et d'une seconde population de patients ayant un cancer mais non un cancer récidivant et au-dessous duquel il serait attendu qu'entre 95 % et 100 % des patients seraient dans ladite seconde population de patients ;
- établir un second seuil dudit biomarqueur déterminé sur la base d'une plage de taux dudit biomarqueur obtenue à partir d'une première population de patients ayant un cancer primaire et un cancer récidivant et d'une seconde population de patients ayant un cancer mais non un cancer récidivant au-dessus duquel il serait attendu qu'entre 95 % et 100 % des patients seraient dans ladite première population de patients ;
- obtenir un taux d'un biomarqueur chez un patient qui a préalablement été traité pour le cancer par mesure du taux de ce biomarqueur dans un échantillon provenant du patient ;
- appliquer ledit taux pour déterminer si ledit taux est ou non au-dessous dudit premier seuil, indiquant qu'aucune intervention de diagnostic courante n'est requise ou si ledit taux est ou non au-dessus dudit second seuil, indiquant qu'une intervention de diagnostic augmentée est requise ; et
- déterminer que ledit patient nécessite un régime de diagnostic conforme à la norme de soin si ledit taux est entre ledit premier seuil et ledit second seuil.

2. Procédé selon la revendication 1, dans lequel ledit biomarqueur est choisi dans le groupe consistant en métalloprotéinases de matrice (MMP), lipocaline associée à la gélatinase neutrophile (NGAL), complexe MMP/NGAL, thymosine βI5, thymosine βI6, produit génique collagène-like (CLG), prohibitine, glutathion-S-transférase, bêta-5-tubuline, ubiquitine, tropomyosine, Cyr61, cystatine B, chaperonine 10, TIMP et profiline.

3. Procédé selon la revendication 2, dans lequel ladite MMP est choisie dans le groupe consistant en MMP-2, MMP-9, complexe MMP9/NGAL, complexe MMP/TIMP, complexe MMP/TIMP1, ADAMTS-7 ou ADAM-12.
